# EUROPÄISCHE PATENTANMELDUNG

(11) **EP 3 028 741 A1**
(43) Veröffentlichungstag der Anmeldung: **08.06.2016**
(21) Anmeldenummer: 15193416.3
(22) Anmeldetag: 06.11.2015
(51) Int. Cl.: A61N 1/375, H01R 13/187

(54) **KONTAKTELEMENT UND VERFAHREN ZUR HERSTELLUNG EINES KONTAKTELEMENTS**

(30) Priorität: 04.12.2014 US 201462087269 P
(71) Anmelder: BIOTRONIK SE & Co. KG, 12359 Berlin (DE)
(72) Erfinder: Starke, Marcel, 15732 Eichwalde (DE); Bohmeyer, Martin, 15366 Neuenhagen (DE); Mannhaupt, Ringo, 14943 Luckenwalde (DE); Rebentisch, Ronald, 10967 Berlin (DE); Oertmann, Torsten, 15827 Blankenfelde (DE)
(74) Vertreter: Galander, Marcus

(57) **Zusammenfassung**

Die Erfindung betriff eine Federhülse (10) für ein elektrisches Kontaktelement (30), insbesondere für ein medizinisches Implantat, umfassend eine Steckerbohrung (16) zur Aufnahme eines elektrischen Gegenkontakts (110) in einer Einführrichtung (12); wenigstens einen zumindest bereichsweise um die Steckerbohrung (16) angeordneten und zur Steckerbohrung (16) wenigstens bereichsweise offenen Federaufnahmebereich (40), der zur Aufnahme eines Federelement (52) zur elektrischen Kontaktierung des elektrischen Gegenkontakts (110) vorgesehen ist, wobei ein Freiraum (22) im Federaufnahmebereich (40) vorgesehen ist, so dass ein bestimmungsgemäß eingesetztes Federelement (52) bei eingestecktem Gegenkontakt (110) in den Freiraum (22) ausweichen kann.

Ferner wird ein elektrisches Kontaktelement (30) mit einer Federhülse vorgeschlagen, sowie ein Verfahren zur Herstellung eines Kontaktelements (30).

## Beschreibung

Die Erfindung betrifft ein elektrisches Kontaktelement und ein Verfahren zur Herstellung eines elektrischen Kontaktelements nach den Oberbegriffen der unabhängigen Ansprüche.

Medizinische Implantate, insbesondere aktive implantierbare medizinische Geräte, die in den menschlichen oder tierischen Körper einführbar sind und die eine elektrische Versorgungsspannung benötigen, erfordern eine besonders sichere und dauerhafte elektrische Kontaktierung. Solche Implantate sind beispielsweise Herzschrittmacher, Defibrillatoren, Neuro-Stimulatoren und dergleichen.

Die EP 2 614 856 Al offenbart eine Kontaktvorrichtung mit einem Kontaktelement und einem Gegenkontakt, der in eine Steckerbohrung des Kontaktelements eingesteckt wird. Dabei kommen im Kontaktelement angeordnete Drahtwendeln mit elektrischen Kontaktflächen des Gegenkontakts in Berührung. Die Drahtwendeln wirken radial auf die Kontaktflächen, und werden in entsprechenden passgenauen Aussparungen des Kontaktelements radial nach außen abgestützt.

Aus der DE 690 27 846 T2 ist ein elektrisches Kontaktelement für in den menschlichen oder tierischen Körper einführbare medizinische Implantate bekannt, bei dem eine elektrische Verbindung über eine Torusfeder und einen Pin eines Steckkontakts hergestellt wird. Die Torusfeder ist in eine Nut einer Buchse eingelegt, wobei der elektrische Kontakt zwischen dem Pin und dem Innenumfang der Torusfeder ausgebildet ist.

Die Fertigung von Torusfedern, insbesondere geschlossener Torusfedern, und deren Montage durch Einführen in die umlaufende Nut entsprechender Federhülsen ist aufwendig, schwer automatisierbar und daraus resultierend entsprechend kostenintensiv.

Der Erfindung liegt die Aufgabe zugrunde, eine Federhülse für ein elektrisches Kontaktelement zu schaffen, die zumindest weitgehend automatisiert herstellbar ist.

Der Erfindung liegt die Aufgabe zugrunde, ein elektrisches Kontaktelement zu schaffen, das zumindest weitgehend automatisiert herstellbar ist.

Weiterhin soll ein Verfahren zur Herstellung eines solchen Kontaktelements geschaffen werden.

Die Aufgaben werden erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Nach einem Aspekt der Erfindung wird eine Federhülse für ein elektrisches Kontaktelement vorgeschlagen, insbesondere für ein medizinisches Implantat, umfassend
- eine Steckerbohrung zur Aufnahme eines elektrischen Gegenkontakts in einer Einführrichtung,
- wenigstens einen zumindest bereichsweise um die Steckerbohrung angeordneten und zur Steckerbohrung wenigstens bereichsweise offenen Federaufnahmebereich, der zur Aufnahme eines Federelements zur elektrischen Kontaktierung des elektrischen Gegenkontakts vorgesehen ist. Es ist wenigstens einen Freiraum im Federaufnahmebereich vorgesehen, so dass ein bestimmungsgemäß eingesetztes Federelement bei eingestecktem Gegenkontakt in den Freiraum ausweichen kann.

Als medizinische Implantate sind im Sinne der Erfindung insbesondere aktive implantierbare medizinische Geräte zu verstehen, wie z.B. Herzschrittmacher, elektrische Neurostimulatoren und dergleichen.

Vorteilhaft kann die Federhülse bei der Herstellung mit größeren Toleranzen gefertigt werden als bei einer rein radial wirkenden Feder nach dem Stand der Technik, da bei eingesetztem Federelement der Federaufnahmebereich das Federelement nicht radial außen abstützen muss. Dadurch sind preisgünstige Herstellverfahren für die Federhülse einsetzbar, wie etwa urformende Sinterverfahren. Die Kontaktkraft des Federelements wird nicht durch radiale Vorspannung zwischen Gegenkontakt, Federelement und Federhülse erreicht, sondern kann durch Erzeugen einer Zugspannung im Federelement erreicht werden.

Gemäß einer günstigen Ausgestaltung kann der Federaufnahmebereich im Wesentlichen U-förmig ausgebildet sein, mit einem Bogenabschnitt und vom Bogenabschnitt abgehende Kanälen. Dies ermöglicht eine günstige Fertigung der Federhülse wie auch eines einzusetzenden Federelements, das als Zugschlaufe ausgebildet werden kann. Der Bogenabschnitt umgibt dabei wenigstens bereichsweise die Steckerbohrung und weist radial nach außen einen Freiraum für das Federelement auf. Die abgehenden Kanäle können zur Fixierung des Federelements dienen. Die Kanäle können nach außen offen sein, so dass bei eingesetztem Federelement eine Fixierung und/oder eine elektrische Kontaktierung des Federelements möglich ist.

Alternativ kann der Federaufnahmebereich so ausgebildet sein, dass dieser zwei oder mehr Federelementstücke aufnehmen kann, etwa parallele Stücke, sich kreuzende Stücke und dergleichen.

Gemäß einer günstigen Ausgestaltung kann die Federhülse wenigstens zweiteilig ausgebildet sein, mit einem ersten Hülsenteil und einem zweiten Hülsenteil, die fest miteinander verbindbar sind. Dies erlaubt eine günstige Fertigung der Federhülse und eine günstige Fertigung des Federaufnahmebereichs in einem der Hülsenteile, der mit dem anderen Hülsenteil verschlossen werden kann. Alternativ kann jeweils ein Teil des Federaufnahmebereichs in den Hülsenteilen eingebracht werden, so dass der Federaufnahmebereich erst bei zusammengesetzter Federhülse vollständig ausgebildet ist. Vorteilhaft kann der Federaufnahmebereich außerhalb einer Trennebene der wenigstens zwei Hülsenteile liegen, so dass der Federaufnahmebereich im Grundkörper ausgebildet ist, der durch einen Deckel verschlossen wird. Durch eine derartige asymmetrische Aufteilung in Grundkörper und Deckel ergeben sich vielfältige und kostengünstige Gestaltungsmöglichkeiten mit urformenden, umformenden oder auch spanenden Herstellmethoden. Der Federaufnahmebereich kann mit großer Gestaltungsfreiheit ausgeführt werden. Ebenso können die Hülsenteile einfach zueinander positioniert und miteinander gefügt werden.

Nach einem weiteren Aspekt der Erfindung wird ein elektrisches Kontaktelement vorgeschlagen, insbesondere für ein medizinisches Implantat zum Einführen in den menschlichen oder tierischen Körper, umfassend:
- eine Federhülse mit einer Steckerbohrung zur Aufnahme eines elektrischen Gegenkontakts in einer Einführrichtung, wobei die Federhülse wenigstens einen zumindest bereichsweise um die Steckerbohrung angeordneten und zur Steckerbohrung wenigstens bereichsweise offenen Federaufnahmebereich aufweist;
- wenigstens ein in dem Federaufnahmebereich angeordnetes Federelement zur elektrischen Kontaktierung des elektrischen Gegenkontakts in einem eingesteckten Zustand des Gegenkontakts. Es ist vorgesehen, dass der Federaufnahmebereich bei nicht eingestecktem Gegenkontakt einen Freiraum aufweist, in den das Federelement bei in die Steckerbohrung eingestecktem Gegenkontakt ausweicht.

Als medizinische Implantate sind im Sinne der Erfindung insbesondere aktive implantierbare medizinische Geräte zu verstehen, wie z.B. Herzschrittmacher, elektrische Neurostimulatoren und dergleichen.

Dadurch, dass ein Freiraum in dem Federaufnahmebereich vorgesehen ist, kann das Federelement bei eingestecktem Gegenkontakt gedehnt und auf Zugspannung beansprucht werden, wobei sich eine günstige und stabile Multikontaktanlage des Federelements am Gegenkontakt bildet. Vorteilhaft kann das Federelement länglich ausgebildet sein, insbesondere als Drahtwendel. Vorteilhaft kann die Federhülse für jedes der Federenden des länglichen Federelements je einen Kanalabschnitt im Federaufnahmebereich aufweisen. Der Federaufnahmebereich umfasst vorteilhaft einen Bogenabschnitt, der teilweise um die Steckerbohrung angeordnet ist, sowie davon abgehende Kanäle. Der Federaufnahmebereich ist so ausgebildet, dass das Federelement einerseits einen möglichst großen Umschlingungswinkel am Gegenkontakt ausbilden kann, vorzugsweise mindestens 180°, und andererseits für das Federelement einen ausreichenden Freiraum nach außen bietet, um die Federwirkung und/oder die Federverformung aufbringen zu können. der Federaufnahmebereich ist so ausgeführt, dass das Federelement in entspannter Lage, d.h. ohne eingesteckten Gegenkontakt, in die Steckerbohrung eingreifen kann. Das Federelement weist ohne Gegenkontakt im Bogenabschnitt einen Innendurchmesser auf, der geringer ist als der Außendurchmesser des Gegenkontakts, dehnt sich beim Einstecken des Gegenkontakts und kann in den Freiraum des Federaufnahmebereich um die Steckerbohrung radial nach außen ausweichen.

Nach einer günstigen Ausgestaltung kann das Federelement an der Federhülse so festgelegt sein, dass das Federelement beim Ausweichen in den Freiraum eine Zugfeder bildet. Sowohl das Federelement als auch die Federhülse können daher mit vorteilhaften Toleranzen hergestellt werden, da geringere Ansprüche an eine formgenaue Anpassung von Federhülse, Federaufnahmebereich und Federelement erfüllt werden müssen als bei einer nur radial wirkenden Abstützung des Federelements.

Das Federelement kann als Federwendel ausgebildet sein, bei der ein Draht zu einer Wendel aufgewickelt ist. Der Draht kann zur Verbesserung des Kontaktwiderstands zum Gegenkontakt einen beliebigen Querschnitt aufweisen, etwa rund, eckig, sternförmig etc. Vorteilhaft kann das Federelement aus einem geeigneten, insbesondere biokompatiblen, Material gebildet sein, das hinreichend gute Federeigenschaften aufweist. Einige Beispiele für solche Materialien sind eisenbasierte Legierungen, wie z.B. 316, Titanlegierungen, wie z.B. TiA16V4, eisenfreie Legierungen, wie z.B. MP35N, oder Platinlegierungen, wie z.B. PtIr und weitere Platinlegierungen mit speziellen Federeigenschaften, die wenigstens ein Element aus der Gruppe Palladium, Kupfer, Molybdän, Wolfram, Niob, Tantal enthalten.

Platinlegierungen mit Federeigenschaften, die für diesen Einsatzzweck besonders geeignet sind, sind beispielsweise aus der DE 37 12 839 C1 bekannt. Diese Platinlegierungen mit hoher Härte und guter Verformbarkeit behalten auch nach einer Wärmeeinwirkung, etwa beim Schweißen oder Löten, ihre Federeigenschaften.

Denkbar ist auch die Verwendung eines Kernmaterials im Inneren des Federelements mit guten Federeigenschaften, das mit einer Beschichtung mit guter Körperverträglichkeit und guter elektrischer Leitfähigkeit versehen ist, etwa einem Federstahl oder MP35N als Kernmaterial mit einer Beschichtung aus Pt, PtIr, Au, Pd oder dergleichen. MP35N ist eine aushärtbare Legierung auf Nickel-Kobalt-Basis.

Die Beschichtung kann dabei für den Einsatz hinreichende Federeigenschaften aufweisen und/oder eine für den Einsatz hinreichende elektrische Leitfähigkeit. Die Beschichtung kann chemisch, elektrochemisch, physikalisch - z.B. durch Plattieren oder Kaltverschweißen - in einer oder in mehreren Schichten erfolgen.

Es ist vorteilhaft, wenn die Federelemente und/oder Federhülsen biokompatibel ausgebildet sind. Da diese jedoch nicht im direkten Körperkontakt stehen, ist Biokompatibilität keine unabdingbare Eigenschaft. Besonders vorteilhaft ist, wenn Federelemente und/oder Federhülsen stabil gegen Korrosion und/oder elektrochemische Belastung in der Umgebung von Körperflüssigkeiten, wie z.B. Blut, ausgebildet und/oder mit einer entsprechenden Beschichtung versehen sind.

Zweckmäßigerweise kann das Material für den Einsatz hinreichende Federeigenschaften und eine für den Einsatz hinreichende elektrische Leitfähigkeit aufweisen.

Ferner kann ein formglühbares Material eingesetzt werden, um Feder-Formelemente herzustellen, wie etwa MP35N. Das Feder-Formelement kann bereits in seiner Gestalt an den Federaufnahmebereich angepasst sein, wodurch bei der Montage des Federformelements in die Federhülse und auch bei Funktion als Feder-Kontaktelement Vorteile entstehen.

Das Federelement dient zumindest an den zur Steckerbohrung offenen Bereichen des Federaufnahmebereichs als elektrische Kontaktfläche für den in die Steckerbohrung eingesteckten Gegenkontakt, und liegt mit Federkraft an dem Gegenkontakt an. Zweckmäßigerweise liegt der Federaufnahmebereich in einer konstanten axialen Höhe bezogen auf die Längsachse der Steckerbohrung. Denkbar ist jedoch auch, dass der Federaufnahmebereich eine Steigung aufweisen kann. Vorteilhaft kann der Federaufnahmebereich U-förmig ausgebildet sein.

Vorteilhaft können die Kanäle schräg oder senkrecht zum Bogenabschnitt des Federaufnahmebereichs angeordnet sein. Damit ist es möglich, dass das Federelement einen großen Teil eines eingesteckten Gegenkontakts umschlingen und sich eine große Kontaktfläche bilden kann.

Das Federelement kann bei nicht eingestecktem Gegenkontakt wenigstens teilweise in die Steckerbohrung hineinragen, womit eine sichere Kontaktierung des Gegenkontakts durch Ausbilden einer Zugspannung im Federelement sichergestellt ist.

Dabei ist in einer zweckmäßigen Ausgestaltung vorgesehen, dass das Federelement bei nicht eingestecktem Gegenkontakt im Bereich des Bogenabschnitts einen lichten Innendurchmesser aufweist, der geringer ist als ein Außendurchmesser des bestimmungsgemäßen Gegenkontakts. Der Unterschied der Durchmesser kann gering sein, der Innendurchmesser des Federelements kann geringfügig kleiner sein als der Außendurchmesser des Gegenkontakts.

Besonders vorteilhaft kann das Federelement als Zugschlaufe ausgebildet sein, um bei eingestecktem Gegenkontakt diesen mindestens um 180° an seinem Außenumfang zu umschlingen. Damit ist eine stabile elektrische Kontaktierung zwischen Federelement und Gegenkontakt möglich. Denkbar ist auch ein rundes Federelement, das in dem Bogenabschnitt des Federaufnahmebereichs der Federhülse liegt, wobei das runde Federelement lose darin liegen kann oder z.B. an einem oder mehreren Punkten an der Federhülse fixiert sein kann.

In einer Ausgestaltung kann das freie Ende des Federelements in dem einen Kanal des Federaufnahmebereichs eingelegt sein und durch einen zweiten Kanal, der parallel zu ersten liegt, am Umfang der Federhülse herausgeführt sein, um einen wenigstens teilweise umlaufenden Federkontakt herzustellen. Das Federelement kann auch ein nicht komplett geschlossenes Torusfederelement sein, das beispielsweise in dem ersten Kanal des Uförmigen Federaufnahmebereichs, dem Bogenabschnitt und in einem zusätzlichen weiteren, tangential zum Bogenabschnitt verlaufenden Kanal wieder aus dem Bogenabschnitt herausgeführt werden kann. Dieser zweite Kanal kann beispielsweise ungefähr parallel zum ersten Kanal geführt sein.

Gemäß einer günstigen Ausgestaltung kann das Federelement an wenigstens einem Punkt mit der Federhülse fest verbunden sein. Vorteilhaft können beide Federenden mit der Federhülse fest verbunden sein. Bei einer definierten Auslenkung des Federelements kann hierdurch eine definierte Zugspannung erzeugt werden. Vorteilhaft kann das Federelement z.B. im Bereich des Kanals des Federaufnahmebereichs, mit der Federhülse verschweißt sein. Insbesondere kann das Federelement mit der Federhülse laserverschweißt sein, was ein besonders genaues Platzieren des Schweißpunkts erlaubt.

Eine Kontaktvorrichtung kann z.B. mehrere derartige Kontaktelemente mit Federhülsen und Federelementen in einer axialen Richtung aneinandergereiht aufweisen, wobei die umlaufenden Bogenabschnitte der jeweiligen Federaufnahmebereiche konzentrisch, vorzugsweise parallel zueinander angeordnet sind, so dass ein Gegenkontakt mit voneinander elektrisch isolierten Kontaktbereichen mit den jeweiligen Federelementen der jeweiligen Federhülsen in Kontakt kommen kann.

In einer weiteren günstigen Ausgestaltung kann das Federelement aus einem oder beiden der Kanäle des Federaufnahmebereichs herausstehen. Das herausstehende Ende des Federelements kann zur elektrischen Kontaktierung der Federhülse selbst eingesetzt werden. Alternativ kann das Federelement bündig mit dem Kanal abschließen und die Federhülse an anderer Stelle elektrisch kontaktiert werden.

Gemäß einer günstigen Ausgestaltung kann die Federhülse zweiteilig oder mehrteilig ausgebildet sein. Damit lässt sich das Federelement besonders einfach montieren. Günstig ist es, den Federaufnahmebereich in einem Teil der Federhülse vorzusehen und einen anderen Teil der Federhülse als einfach geformten Deckel vorzusehen. Ferner ist es durch die geteilte Federhülse möglich, den Bogenabschnitt und die Kanäle des Federaufnahmebereichs weitgehend frei und nach Bedarf zu gestalten, wozu urformende, umformende oder auch spanende Verfahren eingesetzt werden können. Weiterhin können die Teile der Federhülse unkompliziert zueinander positioniert und kostengünstig fest miteinander verbunden werden.

Vorteilhaft kann der Federaufnahmebereich außerhalb einer Trennebene der zweiteiligen oder mehrteiligen Federhülse liegen. Der Querschnitt der Federhülse kann beliebig gewählt sein, wie etwa rund, quadratisch etc. Das Federelement kann in den Federaufnahmebereich eingelegt werden, an der Federhülse befestigt werden, insbesondere im Bereich der Kanäle des Federaufnahmebereichs, und mit dem Deckel verschlossen werden.

Vorteilhaft kann das Federelement eine Drahtwendel sein. Diese bietet besonders zahlreiche Kontaktpunkte im Kontakt mit dem Gegenkontakt. Die Drahtwendel kann ähnlich hergestellt werden wie Wendeln für Herzschrittmacherelektrodenzuleitungen, d.h. eine solche Wendel kann aus einem Draht oder mehreren einzelnen Drähten gewickelt und in größeren Längeneinheiten vorgefertigt werden. Dies erlaubt eine besonders wirtschaftliche Herstellung des Federelements.

Ferner wird ein Verfahren zur Herstellung eines Kontaktelements vorgeschlagen, wobei ein Federelement mit einer gewünschten Länge in einen Federaufnahmebereich einer Federhülse gelegt wird, mit der Federhülse fest verbunden wird und der Federaufnahmebereich mit einem Hülsenteil der Federhülse verschlossen wird. Dies erlaubt eine wirtschaftliche Herstellung eines Kontaktelements.

Das Federelement kann günstigerweise formgeglüht werden, um eine Form in der Art des Federaufnahmebereichs zu erreichen.

Mit besonderem Vorteil kann eine Kontaktkraft des Federelements voreingestellt werden, indem ein Test-Gegenkontakt in die Steckerbohrung gesteckt wird und das Federelement bei gestecktem Test-Gegenkontakt mit einer definierten Zugkraft beaufschlagt und in diesem Zustand mit der Federhülse fest verbunden wird und gegebenenfalls abgelängt wird.

Vorteilhaft kann das Federelement eine Drahtwendel sein. Derartige Drahtwendeln lassen sich in großen Längen herstellen und können passend zugeschnitten werden.

Vorteilhaft sind Kosteneinsparungen durch einen Herstellungsprozess mit hoher Automatisierbarkeit möglich. Die Drahtwendel kann direkt aus dem Wickelprozess über ein Führungselement über einen Durchführkanal in die Nut der Federhülse laufen.

Das eingeführte Federelement kann als Verdrahtungselement genutzt und an einem Punkt automatisch mit der Federhülse verschweißt und in entsprechender Länge getrennt werden. Alternativ kann das Federelement mit der Federhülse bündig verschweißt und abgelängt werden.

Die Erfindung ist nachfolgend beispielhaft, anhand von in Zeichnungen dargestellten Ausführungsbeispielen, näher erläutert. Es zeigen in schematischer Darstellung:
- Fig. 1: eine schematische Darstellung einer Kontaktvorrichtung mit Kontaktelementen nach einer ersten Ausgestaltung der Erfindung;
- Fig. 2: eine Ansicht einer ersten Ausgestaltung einer Federhülse mit auf einen Bogenabschnitt zulaufende Kanäle eines Federaufnahmebereichs in der Federhülse;
- Fig. 3: einen Schnitt durch die Federhülse aus Figur 2;
- Fig. 4: eine Draufsicht auf eine erste Ausgestaltung eines Federaufnahmebereichs in einer Federhülse;
- Fig. 5: eine Draufsicht auf eine weitere Ausgestaltung eines Federaufnahmebereichs in einer Federhülse;
- Fig. 6: eine Draufsicht auf eine weitere Ausgestaltung eines Federaufnahmebereichs in einer Federhülse;
- Fig. 7: eine teilaufgeschnittene Federhülse mit in einen Federaufnahmebereich eingelegtem Federelement;
- Fig. 8: schematisch eine elektrische Kontaktfläche eines Gegenkontakts in Kontakt mit einer Federhülse;
- Fig. 9: eine günstige Ausgestaltung einer Federhülse mit Federelement;
- Fig. 10: eine günstige Ausgestaltung einer Federhülse mit zwei Federelementen;
- Fig. 11: eine günstige Ausgestaltung einer Federhülse mit drei Federelementen;
- Fig. 12a-12c: Beispiele von langgestreckten Drahtwendeln; und
- Fig. 13a-13c: Beispiele von ringförmigen Drahtwendeln aus Figur 12a-12c.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

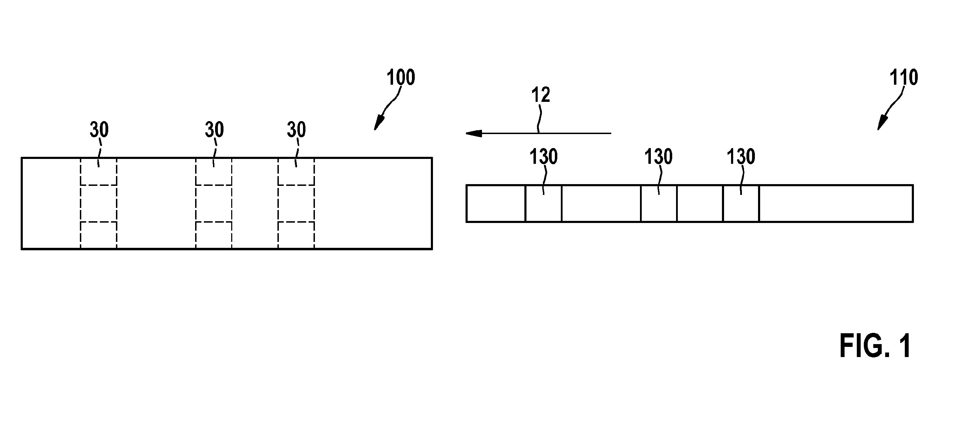

Figur 1 zeigt eine schematische Ansicht einer Ausgestaltung einer Kontaktvorrichtung 100 mit einer Mehrzahl, beispielsweise drei, von elektrischen Kontaktelementen 30. Details eines medizinischen Implantats, insbesondere eines aktiven medizinischen Geräts, mit dem die Kontaktvorrichtung 100 und ein dieser zugeordneter elektrischer Gegenkontakt 110 gekoppelt sind, wie z.B. angeschlossene Komponenten, elektrische Bauteile und dergleichen, sind nicht dargestellt.

Der elektrische Gegenkontakt 110, beispielsweise ein Pin, ist dazu vorgesehen, in Richtung 12 einer Längsachse in die Kontaktvorrichtung 100 eingesteckt zu werden. Dabei wird der elektrische Gegenkontakt 110 durch die Kontaktelemente 30 durchgeführt. Der elektrische Gegenkontakt 110 weist an seiner Außenseite eine Mehrzahl, beispielsweise drei, von Kontaktflächen 130 auf, die mit den Kontaktelementen 30 in Kontakt kommen, wenn der elektrische Gegenkontakt 110 in die Kontaktvorrichtung 100 eingesteckt ist.

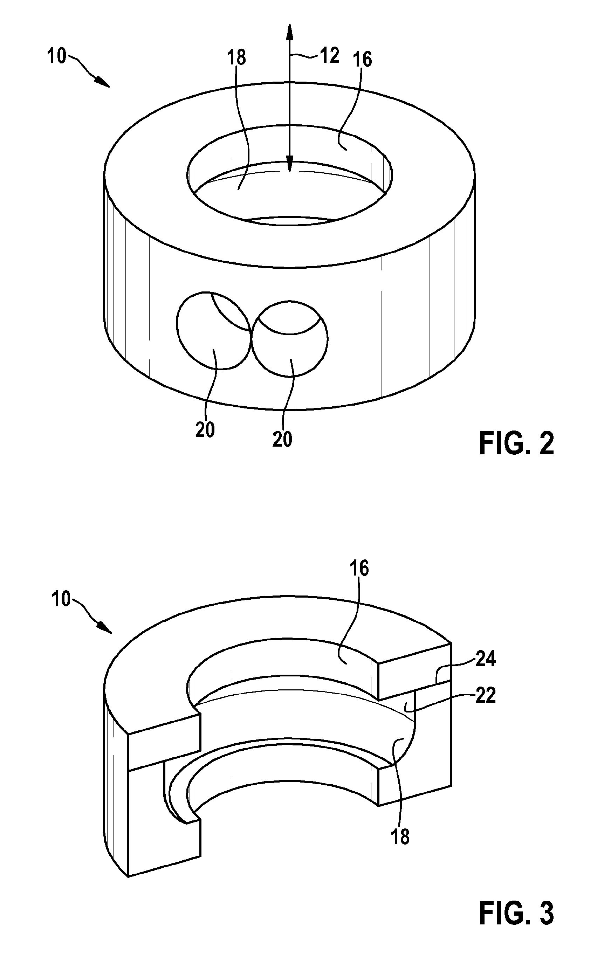

Die Figuren 2 und 3 zeigen zur Erläuterung der Erfindung eine isometrische Ansicht einer Federhülse 10 eines Kontaktelements 30 (Figur 1) sowie einen Schnitt durch die Federhülse 10 einer ersten Ausgestaltung mit einer Steckerbohrung 16 zur Aufnahme eines elektrischen Gegenkontakts (nicht dargestellt) in einer Einführrichtung 12.

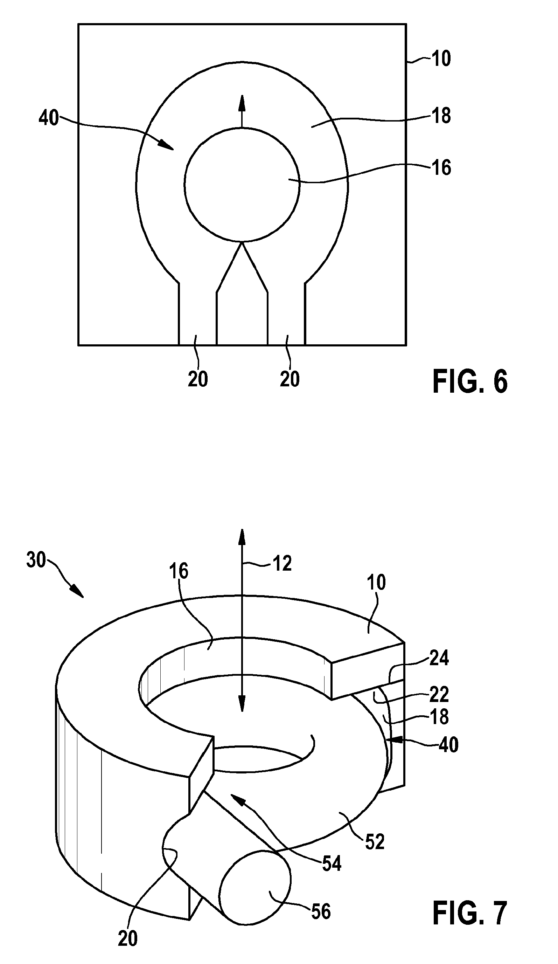

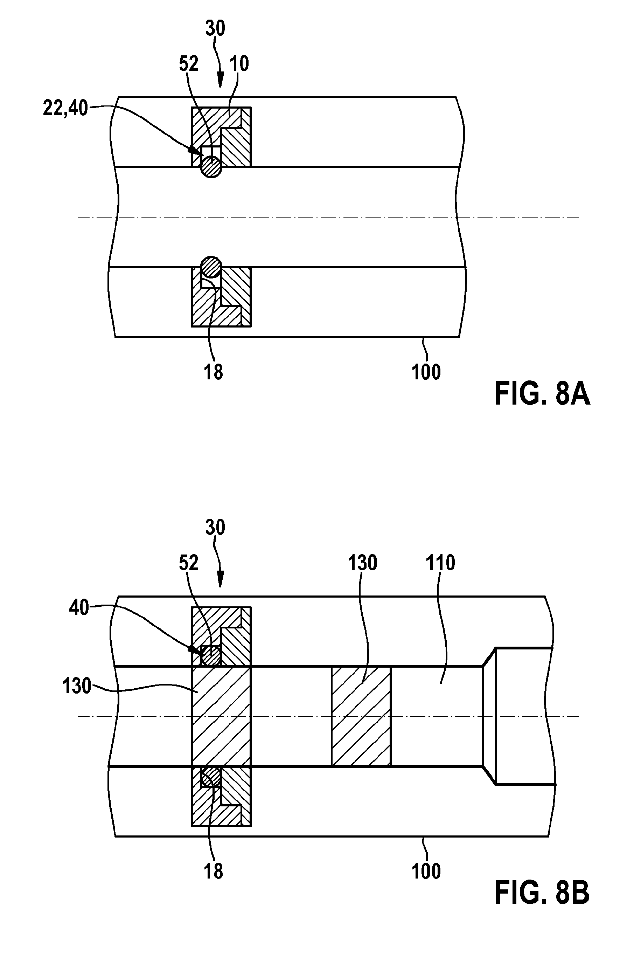

Die Federhülse 10 weist einen Federaufnahmebereich 40 auf. Um die sich in axialer Richtung 12 erstreckende Steckerbohrung 16 ist ein um die Steckerbohrung 16 umlaufende und zur Steckerbohrung 16 offener Bogenabschnitt 18 des Federaufnahmebereichs 40 angeordnet, welcher in einer Ebene quer zur Richtung 12 liegt. Der Bogenabschnitt 18 ist dazu vorgesehen, ein Federelement 52 (Figuren 7, 8) aufzunehmen, um den elektrischen Gegenkontakt (nicht dargestellt) elektrisch zu kontaktieren. Mit dem Federelement 52 (Figuren 7, 8a, 8b) zusammen bildet die Federhülse 10 das Einführelement 30 (Figur 1).

Quer zum umlaufenden Bogenabschnitt 18 in der Federhülse 10 sind zwei Kanäle 20 des Federaufnahmebereichs 40 zur Zuführung des Federelements 52 (Figuren 7, 8a, 8b) in die umlaufende Nut 18 angeordnet. Die Kanäle 20 laufen in etwa parallel von der Außenseite der Federhülse 10 auf den Bogenabschnitt 18 zu und sind in derselben Ebene wie der Bogenabschnitt 18 angeordnet. Der Bogenabschnitt 18 kann z.B. ein prismatisches (eckiges) Profil aufweisen. Bogenabschnitt 18 und Kanäle 20 bilden den Federaufnahmebereich 40 der Federhülse 10.

Für jedes Federende ist ein Kanal 20 eines nicht geschlossenen Federelements 52 (Figuren 7, 8a, 8b) vorgesehen, das von einer Außenseite der Federhülse 10 zum wenigstens einen Bogenabschnitt 18 geführt ist, wobei der Bogenabschnitt 18 bei nicht eingestecktem Gegenkontakt 110 einen radialen Freiraum 22 aufweist, in den das Federelement 52 (Figuren 7, 8a, 8b) bei eingestecktem Gegenkontakt 110 ausweichen kann (Figur 8a, 8b). Dadurch kann eine Zugfederfunktion des Federelements 52 realisiert werden, das durch einen eingeführten Gegenkontakt aufgeweitet werden und in den Freiraum 22 des Bogenabschnitts 18 ausweichen kann. Der Bogenabschnitt 18 braucht dabei wegen des Freiraums 22 das Federelement 52 bei nicht eingestecktem Gegenkontakt nicht nach radial außen abzustützen, sondern nimmt dieses lose auf. Vorteilhaft kann das Federelement 52 (Figuren 7, 8a, 8b) als Drahtwendel ausgebildet sein.

In der hier dargestellten Ausführung ist die Federhülse 10 zweiteilig aufgebaut. Die Trennung der Federhülse 10 erfolgt in der Ebene 24 quer zur Richtung 12 und verläuft außerhalb (in der Figur oberhalb) der Ebene des Federaufnahmebereichs 40. Dieser ist durch Zusammensetzen der beiden Teile der Federhülse 10 abgedeckt. Durch ihren zweiteiligen Aufbau ist die Federhülse 10 kostengünstig herstellbar und damit besonders wirtschaftlich mittels urformender Verfahren wie Spritzen oder Gießen. Denkbar sind auch andere Herstellverfahren. Das Federelement kann in den Federaufnahmebereich 40 des einen Teils der Federhülse 10 eingelegt werden und an dieser fixiert werden, etwa mittels Laserschweißen. Sodann kann das erste Teil mit dem zweiten Teil der Federhülse 10 verschlossen werden.

Die Federhülse 10 ist z.B. zylinderförmig ausgebildet, kann jedoch auch andere Querschnitte aufweisen. Die Kontaktvorrichtung 100 (Figur 1) kann auch mehrere derartige Federhülsen 10 aufweisen, die konzentrisch zur Symmetrieachse und in Richtung 12 aufeinanderfolgend angeordnet sind (nicht dargestellt).

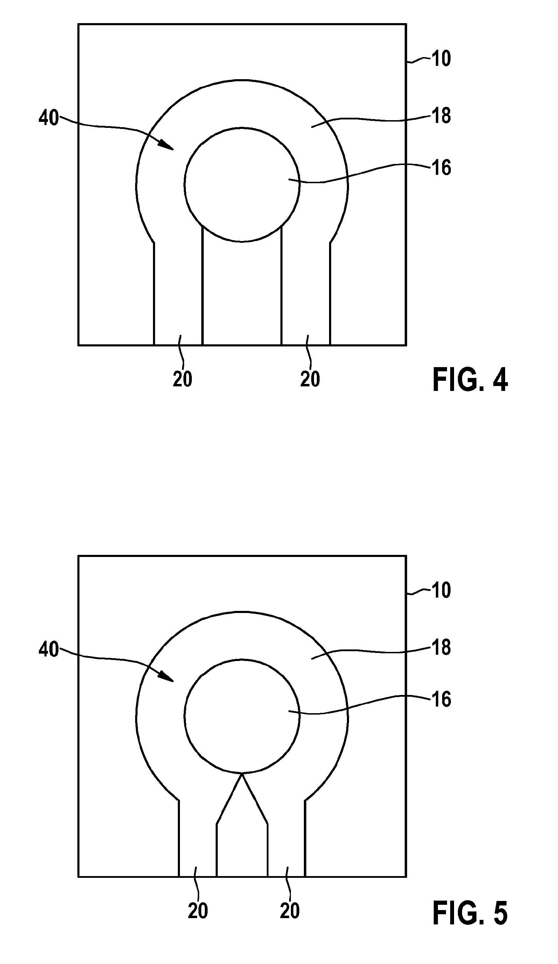

Die Figuren 4 bis 6 zeigen Draufsichten auf verschiedene Ausgestaltungen des Federaufnahmebereichs 40 einer Federhülse 10. Die Federhülse 10 hat in den Ausführungen beispielsweise einen quadratischen Querschnitt, kann jedoch nach Bedarf einen anderen Querschnitt aufweisen. Ein eingesetztes Federelement (nicht dargestellt) ragt zumindest bereichsweise in die Steckerbohrung 16 hinein und wird durch einen eingeführten Gegenkontakt aufgeweitet und in dem Bogenabschnitt 18 des Federaufnahmebereichs 40 radial nach außen gedrückt.

Die Federhülsen 10 in den Figuren sind ähnlich ausgestaltet, so dass zur Vermeidung unnötiger Wiederholungen im Wesentlichen auf die Unterschiede zwischen den Ausgestaltungen eingegangen wird.

Figur 4 zeigt dabei eine Ausgestaltung mit einem Bogenabschnitt 18, auf den beide Kanäle 20 ungefähr tangential zulaufen. In Draufsicht entspricht der Querschnitt des Federaufnahmebereichs 40 in etwa einer U-Form, so dass ein darin eingesetztes Federelement (nicht dargestellt) die Steckerbohrung 16 und damit ein eingeführten Gegenkontakt (nicht dargestellt) als Zugschlaufe umgibt.

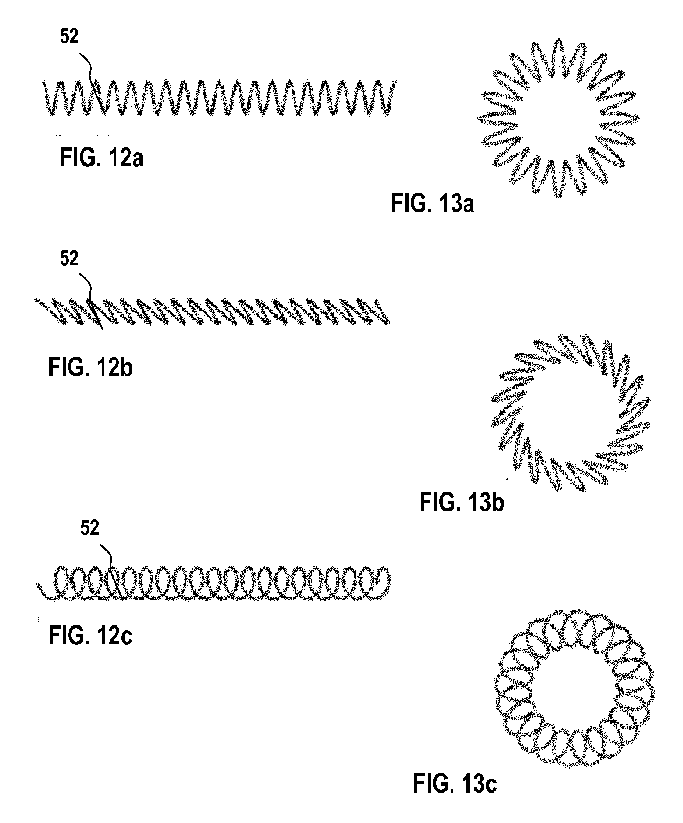

Ein eingesetztes Federelement, beispielsweise in Form eines ursprünglich länglichen Drahtwendelabschnitts (wie beispielsweise in Figuren 12a bis 12c dargestellt), reicht mit einem Federende in den einen der beiden Kanäle 20, umschlingt einen Teil der Steckerbohrung 16 im Bogenabschnitt 18 und reicht mit seinem anderen Federende in den anderen der beiden Kanäle 20. Durch die U-Form des Federaufnahmebereichs wird ermöglicht, dass eine Kontaktkraft des Federelements durch eine Zugfeder in Form einer Schlaufe aufgebracht werden kann und nicht durch radial wirkende Federelemente. Es ergibt sich ein Umschlingungswinkel des Federelements bezogen auf den Gegenkontakt von nahezu 180°.

In mindestens einem der Kanäle 20, vorzugsweise in beiden, ist das Federelement an der Federhülse 10 fixiert, etwa mit einem Schweißpunkt. Durch den Freiraum in dem Bogenabschnitt 18 braucht die Drahtwendel nicht geschränkt zu sein, sondern kann in üblicher Weise radial gewickelt sein, da die Drahtwendel durch die spezielle Konstruktion der Federhülse auf Zug belastet wird. Durch die feste Anbindung des Federelements an die Federhülse 10 ist auch ein elektrischer Kontakt zur Federhülse 10 hergestellt.

Für die Herstellung der Federwendel ist es nicht notwendig, eine besondere Orientierung bei der Montage des Federelements einzuhalten (siehe Figuren 12a und 13a), wie dies bei geschränkten Federelementen der Fall ist (siehe Figuren 12b bis 13c). Dies hat den weiteren Vorteil, dass die Federenden offen bleiben können, da in keiner besonderen Ausrichtung zueinander mit der Federhülse verbunden, insbesondere verschweißt werden können.

Figur 5 zeigt eine Draufsicht auf eine weitere Ausgestaltung eines Federaufnahmebereichs 40 in einer Federhülse 10, bei dem der Federaufnahmebereich 40 so ausgebildet ist, dass für ein darin eingesetztes Federelement ein größerer Umschlingungsbereich eines eingesteckten Gegenkontakts möglich ist. Dabei stoßen die beiden parallel zueinander verlaufenden Kanäle 20 nicht mehr nahezu tangential auf den Bogenabschnitt 18, wie in Figur 4, sondern nahezu radial. Damit ergibt sich ein Umschlingungswinkel eines eingesetzten Federelements bezogen auf den Gegenkontakt von mehr als 180°. Ein eingesetztes Federelement wirkt ebenso wie in Figur 4 als Zugschlaufe.

Bei den Ausgestaltungen in den Figuren 4 und 5 ist der Bogenabschnitt 18 jeweils konzentrisch um die Steckerbohrung 16 ausgebildet. Die Ausgestaltungen eignen sich besonders für formgeglühte Federelemente, die durch das Formglühen bereits eine dem Federaufnahmebereich 40 entsprechende U-Form aufweisen. Der Innendurchmesser des gebogenen Teils des Federelements ist dabei etwas geringer als der Außendurchmesser des Gegenkontakts.

Figur 6 zeigt eine Draufsicht auf eine weitere Ausgestaltung eines aus einem Bogenabschnitt 18 und zwei Kanälen 20 gebildeten Federaufnahmebereichs 40 in einer Federhülse 10, welcher weitgehend dem Federaufnahmebereich 40 in Figur 5 entspricht. Im Gegensatz dazu ist der Bogenabschnitt 18 jedoch exzentrisch zur Steckerbohrung 16 angeordnet. Eine solche Ausgestaltung ist für nichtformgeglühte Federelemente günstig, die bei eingestecktem Gegenkontakt somit in eine radiale Vorzugsrichtung (in der Figur nach oben, wie mit dem Pfeil gekennzeichnet) ausweichen können. Der Federaufnahmebereich 40 führt das Federelement so, dass dieses durch seine Eigenkrümmung im Bogenabschnitt 18 in entspannter Lage in die Steckerbohrung 16 für den Gegenkontakt hineinragt und beim Einstecken des Gegenkontakts nach außen federnd arbeiten kann.

Figur 7 zeigt eine alternative Ausgestaltung mit einer teilweise aufgeschnittenen Federhülse 10 und mit in einen Federaufnahmebereich 40 eingeführtem Federelement 52 mit nur einem zum Bogenabschnitt 18 führenden Kanal 20. Der Bogenabschnitt 18 ist um eine Steckerbohrung 16 konzentrisch umlaufend angeordnet. Wird ein elektrischer Gegenkontakt, z.B. ein Pin (nicht dargestellt), in Richtung 12 in die Steckerbohrung 16 eingeführt, kommt dieser mit dem Federelement 52 in Berührung und stellt eine elektrische Verbindung zwischen Federelement 52 und Gegenkontakt her. Das Federelement 52 ist zur Fixierung seiner Lage an wenigstens einem Punkt mit der Federhülse 10 fest verbunden, z.B. verschweißt. Beim Einstecken des Gegenkontakts wird das Federelement 52 aufgeweitet und weicht in den Freiraum 22 des Bogenabschnitts 18 radial nach außen aus.

Das Federelement 52 ist so weit in den Federaufnahmebereich 40 der Federhülse 10 eingelegt, dass ein vorlaufendes freies Ende des Federelements 52 an einen nachlaufenden Bereich des Federelements 52 anstößt. In der gezeigten Ausgestaltung steht das Federelement 52 aus dem Kanal 20 heraus und kann Anschluss für eine elektrische Kontaktierung der Federhülse 10 verwendet werden. Alternativ kann das Federelement 52 bündig mit dem Kanal 20 abschließen (nicht dargestellt).

Die Figuren 8a und 8b verdeutlichen die Wirkung des Federelements 52 als Zugfeder anhand eines Ausschnitts aus einer Kontaktvorrichtung 100, in dem ein Kontaktelement 30 mit Federhülse 10 und Federelement 52 eingesetzt ist. Figur 8a zeigt die Situation ohne Gegenkontakt, Figur 8b zeigt die Kontaktvorrichtung 100 mit eingestecktem Gegenkontakt 110 mit elektrischen Kontaktflächen 130. Das Federelement kann vorteilhaft als Drahtwendel ausgestaltet sein.

Das Federelement 52 liegt im Federaufnahmebereich 40 der Federhülse 10 und ragt im entspannten Zustand ohne Gegenkontakt 110 in die Steckerbohrung 16 der Federhülse 10. Dabei ist radial um das Federelement ein Freiraum 22 in der Nut 18 vorhanden, so dass das Federelement 52 nicht von der Nut 18 nach außen gestützt ist.

Wird der Gegenkontakt 110 eingesteckt, drängt dieser das Federelement 52 radial nach außen, da der Gegenkontakt 110 der Außendurchmesser geringfügig größer ist als der Innendurchmesser des Federelements 52. Das Federelement 52 kann radial nach außen in den Freiraum 22 ausweichen und dabei als Zugfeder auf den Gegenkontakt wirken.

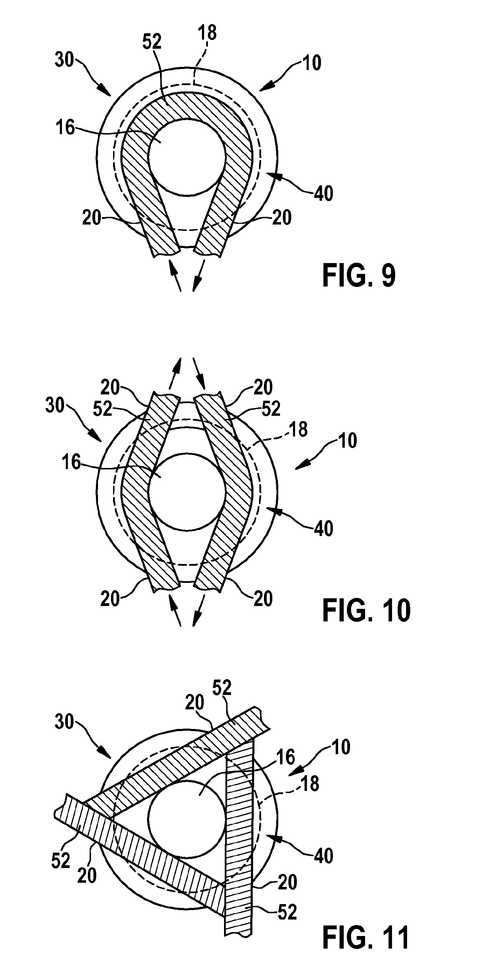

Die Figuren 9, 10 und 11 illustrieren beispielhaft günstige weitere alternative Ausgestaltungen eine Kontaktelements 30 mit einem Federaufnahmebereich 40 mit mehreren Kanälen 20 in der Federhülse 10, die zum Aufnehmen eines mehrteiligen Federelements 52 dienen.

Figur 9 zeigt dabei zwei nebeneinander liegende, schräg zueinander verlaufende Kanäle 20, aus denen die beiden Enden des Federelements 52 herausstehen. Das Federelement 52 kann entsprechend abgelängt werden und vorzugsweise an der Federhülse 10 fixiert werden, wie vorstehend bereits beschrieben wurde. Das Federelement 52 umschließt die Steckerbohrung 16 dabei weitgehend.

Figur 10 zeigt eine günstige Ausgestaltung eines Kontaktelements 30 mit zwei durchgeführten Abschnitten von zwei Federelementen 52, die auf beiden Seiten der Federhülse 10 aus den Kanälen 20 mit ihrem freien Ende mehr oder weniger weit aus der Federhülse 10 herausragen können. Das Federelement 52 kann entsprechend abgelängt werden und vorzugsweise an der Federhülse 10 fixiert werden, wie vorstehend bereits beschrieben wurde. Die beiden Federelemente 52 umschließen die Steckerbohrung 16 bzw. einen elektrischen Gegenkontakt nicht, sondern berühren eine elektrische Kontaktfläche eines Gegenkontakts an zwei diametral gegenüberliegenden Seiten.

Figur 11 zeigt eine günstige Ausgestaltung eines Kontaktelements 30 mit drei um 120° um die Steckerbohrung 16 versetzt angeordneten Federelementen 52, die tangential und im Wesentlichen in gestreckter Form durch separate Kanäle 20 des Federaufnahmebereichs 40 in die Federhülse 10 eingeführt oder eingelegt sind. Durch einen geeigneten Freiraum in der Nut 18 bzw. dem Federaufnahmebereich der Federhülse 10 kann jeweils das Federelement 52 oder die Federelemente 52 radial nach außen in den Freiraum ausweichen, wenn der Gegenkontakt in die Steckerbohrung 16 eingesteckt wird und als Zugfeder auf den Gegenkontakt wirken.

Die Figuren 12a bis 12c zeigen Beispiele für langgestreckte Drahtwendeln für Federelemente 52. Die Figuren 13a bis 13c zeigen beispielhaft ringförmige Drahtwendeln.

Für die Montage der Federelemente 52 in die Federhülsen ist es nicht notwendig, eine besondere Orientierung des Federelements 52 einzuhalten (siehe Figuren 12a und 13a), wie dies bei geschränkten Federelementen der Fall ist (siehe Figuren 12b bis 13c).

## Patentansprüche

1. Federhülse (10) für ein elektrisches Kontaktelement (30), insbesondere für ein medizinisches Implantat, umfassend
- eine Steckerbohrung (16) zur Aufnahme eines elektrischen Gegenkontakts (110) in einer Einführrichtung (12),
- wenigstens einen zumindest bereichsweise um die Steckerbohrung (16) angeordneten und zur Steckerbohrung (16) wenigstens bereichsweise offenen Federaufnahmebereich (40), der zur Aufnahme eines Federelements (52) zur elektrischen Kontaktierung des elektrischen Gegenkontakts (110) vorgesehen ist,
**gekennzeichnet durch**
einen Freiraum (22) im Federaufnahmebereich (40), so dass ein bestimmungsgemäß eingesetztes Federelement (52) bei eingestecktem Gegenkontakt (110) in den Freiraum (22) ausweichen kann.

2. Federhülse nach Anspruch 1, **dadurch gekennzeichnet, dass** der Federaufnahmebereich (40) im Wesentlichen U-förmig ausgebildet ist mit einem Bogenabschnitt (18) und vom Bogenabschnitt (18) abgehenden Kanälen (20).

3. Federhülse nach Anspruch 1 oder 2, **gekennzeichnet durch** eine wenigstens zweiteilige Ausgestaltung mit einem ersten Hülsenteil und einem zweiten Hülsenteil, die fest miteinander verbindbar sind.

4. Federhülse nach Anspruch 3, **dadurch gekennzeichnet, dass** der Federaufnahmebereich (40) außerhalb einer Trennebene (24) der wenigstens zwei Hülsenteile liegt.

5. Elektrisches Kontaktelement (30), insbesondere für ein medizinisches Implantat, umfassend
- eine Federhülse (10) mit einer Steckerbohrung (16) zur Aufnahme eines elektrischen Gegenkontakts (110) in einer Einführrichtung, wobei die Federhülse (10) wenigstens einen zumindest bereichsweise um die Steckerbohrung (16) angeordneten und zur Steckerbohrung (16) wenigstens bereichsweise offenen Federaufnahmebereich (40) aufweist;
- wenigstens ein in dem Federaufnahmebereich (40) angeordnetes Federelement (52) zur elektrischen Kontaktierung des elektrischen Gegenkontakts (110) in einem eingesteckten Zustand des Gegenkontakts (110),
**dadurch gekennzeichnet, dass**
der Federaufnahmebereich (40) um die Steckerbohrung (16) bei nicht eingestecktem Gegenkontakt (110) einen Freiraum (22) aufweist, in den das Federelement (52) bei in die Steckerbohrung (16) eingestecktem Gegenkontakt (110) ausweicht.

6. Kontaktelement nach Anspruch 5, **dadurch gekennzeichnet, dass** das Federelement (52) an der Federhülse (10) so festgelegt ist, dass das Federelement (52) beim Ausweichen in den Freiraum (22) eine Zugfeder bildet.

7. Kontaktelement nach Anspruch 5 oder 6, **dadurch gekennzeichnet, dass** das Federelement (52) bei nicht eingestecktem Gegenkontakt (110) wenigstens teilweise in die Steckerbohrung (16) hineinragt.

8. Kontaktelement nach einem der Ansprüche 5 bis 7, **dadurch gekennzeichnet, dass** das Federelement (52) bei eingestecktem Gegenkontakt (110) diesen mindestens um 180° an seinem Außenumfang zu umschlingt.

9. Kontaktelement nach einem der Ansprüche 5 bis 8, **dadurch gekennzeichnet, dass** das Federelement (52) eine Drahtwendel ist.

10. Kontaktelement nach Anspruch 9, **dadurch gekennzeichnet, dass** das Federelement (52) aus einem korrosionsresistenten und/oder biokompatiblen Material gebildet ist und/oder mit einem korrosionsbeständigen und/oder biokompatiblen Material beschichtet ist.

11. Verfahren zur Herstellung eines Kontaktelements (30) nach einem der Ansprüche 5 bis 10, wobei ein Federelement (52) mit einer gewünschten Länge in einen Federaufnahmebereich (40) einer Federhülse (10) gelegt wird, mit der Federhülse (10) fest verbunden wird und der Federaufnahmebereich (40) mit einem Hülsenteil der Federhülse (10) verschlossen wird.

12. Verfahren nach Anspruch 11, wobei das Federelement (52) formgeglüht wird, um eine Form in der Art des Federaufnahmebereichs (40) zu erreichen.

13. Verfahren nach Anspruch 11 oder 12, wobei das Federelement (52) in der Federhülse (10) mit einer definierten Zugkraft beaufschlagt wird und im beaufschlagten Zustand mit der Federhülse (10) fest verbunden wird.
